# EUROPÄISCHE PATENTSCHRIFT

(11) **EP 1 769 817 B1**
(45) Veröffentlichungstag und Bekanntmachung des Hinweises auf die Patenterteilung: **25.03.2009**
(21) Anmeldenummer: 06017208.7
(22) Anmeldetag: 18.08.2006
(51) Int. Cl.: A61M 15/00

(54) **Pulverinhalator**
Powder inhaler
Inhalateur de poudre

(30) Priorität: 29.09.2005 DE 102005046644
(43) Veröffentlichungstag der Anmeldung: 04.04.2007
(73) Patentinhaber: Braunform GmbH, 79353 Bahlingen (DE)
(72) Erfinder: Beller, Klaus-Dieter, Dr. med. Dipl.-Ing., 79341 Kenzingen (DE)
(74) Vertreter: Goy, Wolfgang

(56) Entgegenhaltungen:
- WO-A-03/075988
- WO-A-20/06066910
- GB-A- 2 354 451
- US-A- 6 123 070

## Beschreibung

Die Erfindung betrifft einen Pulverinhalator nach dem Oberbegriff des Anspruchs 1.

Der erfindungsgemäße treibgasfreie Pulverinhalator dient zur inhalativen Applikation von pulverförmigen, feststofflichen Arzneimitteln (oder anderen Mitteln). Die Pulverinhalatoren setzen dabei das Aerosol durch den Inhalationsvorgang frei, wobei die Energie für die Dispergierung durch den inspiratorischen Fluß gewonnen wird. Die pulverförmige Substanz ist dabei in einem Vorratsbehältnis enthalten. Je nach Art des Pulverinhalators wird der reine Wirkstoff eingesetzt oder der Wirkstoff mit einem Träger (unbedenklicher Hilfsstoff, meistens Laktose oder Glukose) für adhärierte Wirkstoffpartikel. - Der Nachteil bei den bekannten Pulverinhalatoren besteht darin, daß das Pulver nicht optimal deagglomeriert wird und daß der Entleerungsgrad nicht optimal ist.

Die US-A-6 123 070 zeigt in der Ausführungsform, wie sie in den Fig. 9 und 10 dargestellt ist, einen Pulverinhalator mit einem Vorratsbehältnis für das Pulver der eingangs angegebenen Art. Weiterhin ist eine Inhalationseinrichtung in Form eines Mundstücks mit Zuführkanal vorgesehen. Das zu inhalierende Pulver ist in dem Vorratsbehältnis bevorratet. Während des Inhalierens reißt ein durchgänginger Luftstrom das Pulver mit. Hierzu ist vor dem Vorratsbehältnis eine wendelförmige Verwirbelungseinrichtung vorgesehen. Diese besteht aus einem zylinderförmigen Körper, auf dessen Mantelfläche wendelförmige Stege angeordnet sind. Diese Verwirbelungseinrichtung befindet sich im Eingangsluftstrom vor derjenigen Stelle, wo das Pulver durch den Luftstrom mitgerissen wird.

Die GB-A-2 354 451 zeigt einen Inhalator, bei welchem ebenfalls eine Verwirbelungseinrichtung vorgesehen ist. Diese Verwirbelungseinrichtung ist wendelförmig ausgebildet, jedoch nicht stationär im Gehäuse angeordnet. Vielmehr wird die Verwirbelungseinrichtung um eine Achse in eine Drehbewegung versetzt.

Der Erfindung liegt die **Aufgabe** zugrunde, bei einem Pulverinhalator der eingangs angegebenen Art die Verwirbelung der angesaugten Luft zu verbessern.

Die technische **Lösung** ist gekennzeichnet durch die Merkmale im Kennzeichen des Anspruchs 1.

Dadurch ist ein Pulverinhalator geschaffen, bei welchem das Pulver optimal deagglomeriert und der Entleerungsgrad deutlich verbessert ist. Die Grundidee besteht darin, daß die angesaugte Luft im Eingangsluftstrom verwirbelt und die so verwirbelte Luft anschließend dem Pulver zugeführt wird. Durch diese Verwirbelung der Luft wird das Pulver im Luftstrom fein verteilt. Dabei wird der Luftstrom gezielt auf das Pulver geleitet, wobei die Verwirbelungseinrichtung als Strömungsrichter, Verstärker sowie Verdichter wirkt. Dabei ist die Verwirbelungseinrichtung kombiniert wendel-spiral-förmig ausgebildet. Durch diese Form der Verwirbelungseinrichtung ist eine optimale Strömungsrichtung, Verstärkung sowie Verdichtung der angesaugten Luft gewährleistet und es entsteht die gewünschte Verwirbelung und gezielte Zuführung der verwirbelten Luft auf das Pulver. Unter einer Wendel-Spiral-Form ist zu verstehen, daß die Wendel in Strömungsrichtung gesehen schneckenartig konisch zuläuft. Dabei befinden sich die Wendel auf einer Mittelträgerachse

Die Weiterbildung gemäß Anspruch 2 hat den Vorteil, daß von einem Basis-Pulverinhalator ausgegangen wird. In der Luftzuführungsseite wird eine spezielle Verwirbelungseinrichtung eingesetzt, und zwar entsprechend individuell dem zu verabreichendem Pulver. Dies bedeutet, daß für unterschiedliche Pulversorten unterschiedliche Verwirbelungseinrichtungen zur Verfügung stehen und wahlweise in ein und dasselbe Gehäuse eingesetzt werden können.

Eine weitere Weiterbildung schlägt schließlich Anspruch 3 vor. Indem die Verwirbelungseinrichtung in einem Luftzuführungskanal mit den Randbereichen ihrer Wendel dicht anliegt, ist dadurch eine gezielte Strömungsführung des Luftstroms im Eingangsluftbereich in einer kominierten Wendel-Spiral-Form gewährleistet, weil die zugeführte Luft einerseits durch die Wendel und andererseits durch seitliche Kanalbegrenzung geführt wird. Dadurch kann - wie ausgeführt - der Luftstrom exakt auf das Pulver ausgerichtet, verstärkt und verdichtet werden.

Ein Ausführungsbeispiel eines erfindungsgemäßen Pulverinhalators wird nachfolgend anhand der Zeichnungen beschrieben. In diesen zeigt:

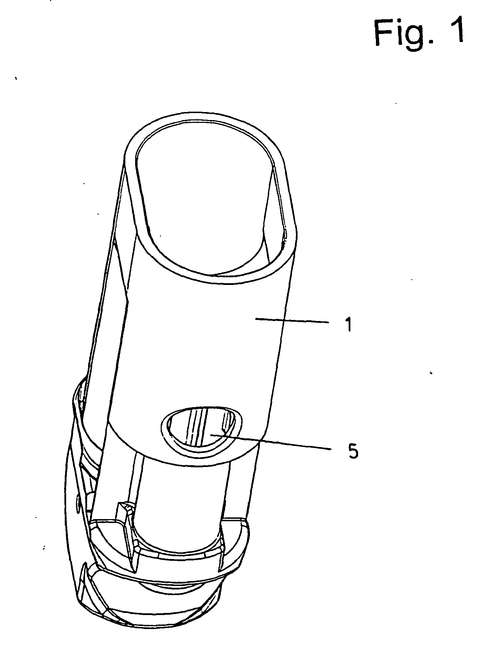

- Fig. 1: eine perspektivische Ansicht des Pulverinhalators;

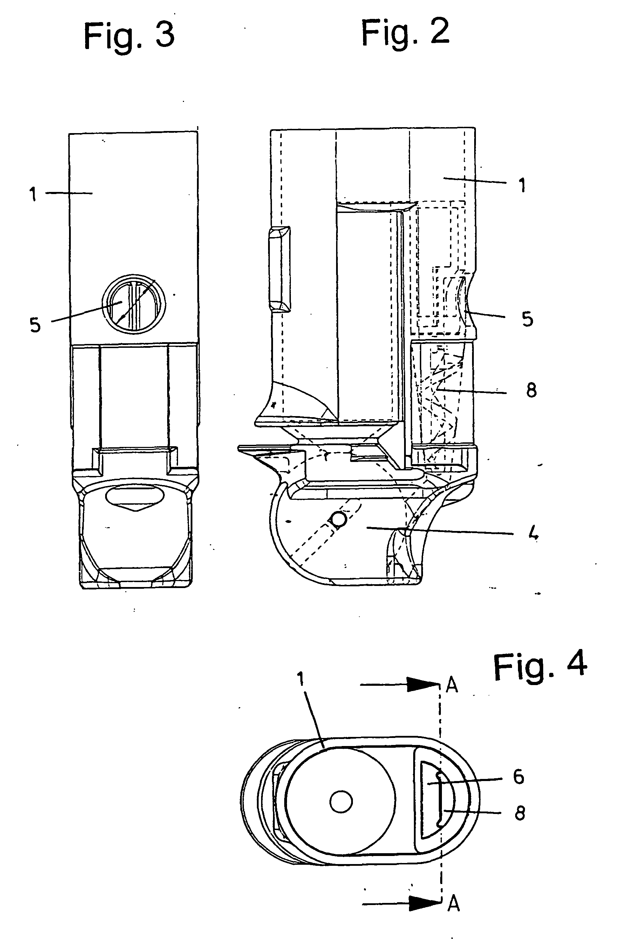

- Fig. 2: eine Seitenansicht des Pulverinhalators in Fig. 1;
- Fig. 3: eine Stirnansicht des Pulverinhalators in Fig. 1;
- Fig. 4: eine Draufsicht auf den Pulverinhalator in Fig. 1;
- Fig. 5: einen Schnitt entlang der Linie A-A in Fig. 4;
- Fig. 6: einen Längsschnitt durch den Pulverinhalator.

Der Pulverinhalator weist ein Gehäuse 1 auf. In diesem ist ein Vorratsbehältnis 2 zur Aufnehme des zu verabreichenden Pulvers ausgebildet. Unterseitig weist dieses Vorratsbehältnis 2 eine Ausgangsöffnung 3 auf. Dieser Ausgangsöffnung 3 schließt sich unterseitig eine Inhalationseinrichtung 4 an.

Das Gehäuse 1 weist weiterhin eine Luftzuführung 5 auf. Diese besitzt einen in Strömungsrichtung trichterförmig sich verjüngenden Kanal 6 auf. Dieser Kanal 6 mündet über eine Zuführungsbohrung 7 in der eigentlichen Inhalationseinrichtung 4.

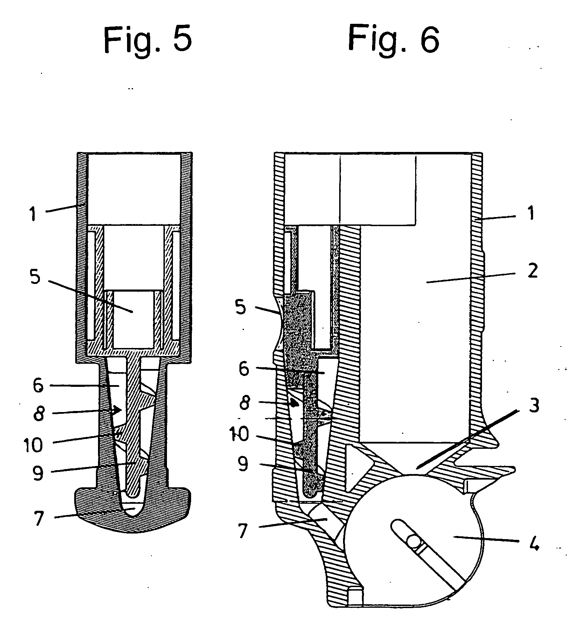

Im Kanal 6 der Luftzuführung 5 befindet sich eine Verwirbelungseinrichtung 8. Diese ist als separates Teil in den Kanal 6 eingesetzt. Die Verwirbelungseinrichtung 8 ist - wie insbesondere aus Fig. 5 und 6 ersichtlich - kombiniert wendel-spiral-förmig ausgebildet. Dies bedeutet, daß eine mittige Achse 9 vorgesehen ist, welche von einer Wendel 10 umschlungen ist, wobei sich diese Wendel 10 in Strömungsrichtung gesehen bezüglich ihres Durchmessers allmählich verringert und dadurch eine Art Spirale definiert. Die Außenmantelfläche der Wendel 10 liegt dabei an der Innenmantelfläche des trichterförmigen Kanals 6 an.

### Die Funktionsweise ist wie folgt:

Aus dem Vorratsbehältnis 2 wird der Inhalationseinrichtung 4 eine vorbestimmte Dosiermenge an Pulver zugeführt.

Anschließend kann der Patient mit der Inhalation beginnen, indem er entweder mit der Nase oder mit dem Mund über die Inhalationseinrichtung 4 das Pulver einsaugt. Dabei ist durch die Luftzuführung 5 mit dem Kanal 6, durch die Zuführungsbohrung 7 und durch die Inhalationseinrichtung 4 ein durchgängiger Luftstrom gebildet, welcher das Pulver während des Inhalierens mitreißt.

Durch die speziell ausgebildete Verwirbelungseinrichtung 8 im Eingangsluftstrom wird dabei die angesaugte Luft verstärkt, verdichtet und so hinsichtlich ihrer Strömung ausgerichtet, daß die Luft exakt auf das Pulver geleitet wird. Dadurch wird das Pulver optimal deagglomeriert und der Entleerungsgrad optimiert.

### Bezugszeichenliste

- 1: Gehäuse
- 2: Vorratsbehältnis
- 3: Ausgangsöffnung
- 4: Inhalationseinrichtung
- 5: Luftzuführung
- 6: Kanal
- 7: Zuführungsbohrung
- 8: Verwirbelungseinrichtung
- 9: Achse
- 10: Wendel

## Patentansprüche

1. Pulverinhalator
mit einem Vorratsbehältnis (2) für das Pulver,
mit einer Inhalationseinrichtung (4) zum Inhalieren einer vorgegebenen Dosierung des Pulvers, wobei während des Inhalierens ein durchgängiger Luftstrom das Pulver mitreißt, sowie
mit einer Verwirbelungseinrichtung (8) in Form einer Wendel (10) für die angesaugte Luft im Eingangsluftstrom vor derjenigen Stelle, wo das Pulver durch den Luftstrom mitgerissen wird,
**dadurch gekennzeichnet,**
**daß** sich die Wendel (10) der Verwirbelungseinrichtung (8) in Strömungsrichtung gesehen bezüglich ihres Durchmessers konisch verringert.

2. Pulverinhalator nach dem vorhergehenden Anspruch,
**dadurch gekennzeichnet,**
**daß** die Verwirbelungseinrichtung (8) ein in den Pulverinhalator einsetzbares, separates Teil ist.

3. Pulverinhalator nach einem der vorhergehenden Ansprüche,
**dadurch gekennzeichnet,**
**daß** die Verwirbelungseinrichtung (8) mit ihrer Umhüllenden in einem komplementären trichterförmigen Kanal (6) des Eingangsluftstroms anliegt.

## Claims

1. Powder inhaler
with a storage container (2) for the powder,
with an inhaler device (4) for inhaling a specific dose of the powder, wherein during inhalation a through air flow carries the powder along; and
with a turbulence device (8) in the form of a helix (10) for the indrawn air in the inlet air flow before the point at which the powder is picked up by the air flow;
**characterised in that**
the helix (10) of the turbulence device (8) tapers conically in diameter viewed in the flow direction.

2. Powder inhaler according to the preceding claim, **characterised in that t**he turbulence device (8) is a separate component which can be inserted in the powder inhaler.

3. Powder inhaler according to any of the preceding claims, **characterised in that** the turbulence device (8) with its sleeve lies in a complementary hopper-shaped channel (6) of the inlet air flow.

## Revendications

1. Inhalateur de poudre
comprenant un récipient (2) de réserve en poudre,
un dispositif inhalateur (4) en vue de l'inhalation d'un dosage préétabli de la poudre, sachant qu'un courant d'air traversant charrie ladite poudre avec lui au cours de l'inhalation,
ainsi qu'un système (8) générateur de tourbillons, revêtant la forme d'une hélice (10) dédiée à l'air aspiré dans le courant d'air d'entrée, avant l'emplacement auquel la poudre est charriée conjointement par ledit courant d'air,
**caractérisé par le fait**
**que** le diamètre de l'hélice (10) du système (8) générateur de tourbillons se rétrécit tronconiquement observé dans la direction de l'écoulement.

2. Inhalateur de poudre selon la revendication précédente,
**caractérisé par le fait**
**que** le système (8) générateur de tourbillons est une pièce distincte pouvant être insérée dans ledit inhalateur de poudre.

3. Inhalateur de poudre selon l'une des revendications précédentes,
**caractérisé par le fait**
**que** le système (8) générateur de tourbillons est en applique, par les extrémités de son enveloppe, dans un canal infundibuliforme complémentaire (6) associé au courant d'air d'entrée.
